# EUROPEAN PATENT APPLICATION

(11) **EP 4 431 112 A1**
(43) Date of publication of application: **18.09.2024**
(21) Application number: 24156163.8
(22) Date of filing: 06.02.2024
(51) Int. Cl.: A61K 41/00

(54) **LYOPHILIZED POWDER CONTAINING BORON COMPLEX AND METHOD OF FORMING THE SAME**

(30) Priority: 14.03.2023 US 202363490004 P; 07.11.2023 TW 112142943
(71) Applicant: Heron Neutron Medical Corp., Zhubei City, Hsinchu County 302 (TW)
(72) Inventor: YU, Yu-Hou, 302 Zhubei City (TW); YU, Chung-Shan, 302 Zhubei City (TW)
(74) Representative: Berggren Oy

(57) **Abstract**

The present disclosure provides a lyophilized powder containing a boron complex. The lyophilized powder includes a sugar acid and a complex formed by a dehydration condensation reaction of a dihydroxyboryl compound and a sugar or a sugar alcohol. The present disclosure also provides a method of forming a lyophilized powder containing a boron complex. The method includes the following operations. A dihydroxyboryl compound and a sugar or a sugar alcohol are mixed to form a mixture, and the mixture includes a complex formed by a dehydration condensation reaction of the dihydroxyboryl compound and the sugar or the sugar alcohol. The mixture is immersed in liquid nitrogen to freeze the mixture to form a pre-frozen body. A vacuum drying process is performed to evaporate water of the pre-frozen body to form a lyophilized powder.

## Description

### BACKGROUND

### Field of Invention

The present disclosure relates to a lyophilized powder containing boron complex and a method of forming the same.

### Description of Related Art

Boron-containing drugs can be used to treat malignancies, such as brain tumors, by applying a boron neutron capture therapy (BNCT). In detail, this technique positions the boron-containing drug to the location of the tumor, and then uses a neutron to irradiate the tumor. The neutron reacts with the ¹⁰B of the boron-containing drug to create radiation that can kill the tumor cells. Compared with other elements in the cells, ¹⁰B has a larger neutron cross section toward the neutron, so the technique kills the cells targeted by the boron-containing drugs with higher selectivity without damaging the surrounding cells. However, an effective boron neutron capture therapy requires a sufficient amount of boron-containing drugs to arrive at the position of the malignancies. The present boron-containing drugs, however, are generally insoluble, which limits the effectiveness of using the drugs, and the drugs may also be hard to store since they easily precipitate from the solution. Therefore, it is necessary to develop a new type of boron-containing drugs and a method of forming the same to effectively improve the solubility of the boron-containing drugs with a formation method that is simple, rapid, and without damaging the boron-containing drugs.

### SUMMARY

The present disclosure provides a lyophilized powder containing boron complex. The lyophilized powder includes a sugar acid and a complex formed by a dehydration condensation reaction of a dihydroxyboryl compound and a sugar or a sugar alcohol.

In some embodiments, the sugar acid includes sialic acid, neuraminic acid, glucuronic acid, mannuronic acid, or combinations thereof.

In some embodiments, the sugar alcohol includes sorbitol, mannitol, xylitol, or combinations thereof.

In some embodiments, the complex includes a first complex formed by the dihydroxyboryl compound and sorbitol and a second complex formed by the dihydroxyboryl compound and mannitol or xylitol.

In some embodiments, when the second complex is formed by the dihydroxyboryl compound and the mannitol, a molar ratio of the first complex to the second complex is from 1:0.25 to 1:1.5; and when the second complex is formed by the dihydroxyboryl compound and the xylitol, a molar ratio of the first complex to the second complex is from 1:0.29 to 1:1.8.

In some embodiments, the lyophilized powder further includes a dihydroxyboryl compound that is not formed in a complex, and in a spectrum of a proton nuclear magnetic resonance, a ratio of an integration of a signal of the complex to an integration of a signal of the dihydroxyboryl compound that is not formed in a complex is from 5 to 25.

In some embodiments, the signal of the complex is from 7.30 ppm to 7.55 ppm, and the signal of the dihydroxyboryl compound that is not formed in a complex is from 7.56 ppm to 7.75 ppm.

In some embodiments, the lyophilized powder further includes a dihydroxyboryl compound that is not formed in a complex, and in a spectrum of a proton nuclear magnetic resonance, the complex includes a signal from 7.00 ppm to 7.19 ppm, and the dihydroxyboryl compound that is not formed in a complex includes a signal from 7.20 ppm to 7.29 ppm.

In some embodiments, a pH value of the lyophilized powder dissolved in water is from 7.4 to 7.8.

In some embodiments, a particle size of the lyophilized powder is from 50 µm to 140 µm.

The present disclosure also provides a method of forming a lyophilized powder containing boron complex. The method includes the following operations. A dihydroxyboryl compound and a sugar or a sugar alcohol are mixed to form a mixture, in which the mixture includes a complex formed by a dehydration condensation reaction of the dihydroxyboryl compound and the sugar or the sugar alcohol. The mixture is immersed into liquid nitrogen to freeze the mixture to form a pre-frozen body. A vacuum drying process is performed to evaporate water from the pre-frozen body to form the lyophilized powder.

in some embodiments, mixing the dihydroxyboryl compound and the sugar or the sugar alcohol further includes mixing a sugar acid to form the mixture, in which the sugar acid includes sialic acid, neuraminic acid, glucuronic acid, mannuronic acid, or combinations thereof.

In some embodiments, mixing the dihydroxyboryl compound and the sugar alcohol further includes mixing a sugar acid to form the mixture, and a weight ratio of an addition amount of the sugar alcohol to an addition of the sugar acid is from 2:1 to 7:1.

In some embodiments, mixing the dihydroxyboryl compound and the sugar or the sugar alcohol further includes mixing a sugar acid to form the mixture, the dihydroxyboryl compound and the sugar or the sugar alcohol are mixed in an alkaline aqueous solution before adding the sugar acid, and a pH value of the alkaline aqueous solution is from 8.3 to 8.7.

In some embodiments, when mixing the dihydroxyboryl compound and the sugar or the sugar alcohol, an equivalent ratio of an addition amount of the sugar or the sugar alcohol to an addition amount of the dihydroxyboryl compound is from 1.0 to 1.5.

In some embodiments, when mixing the dihydroxyboryl compound and the sugar alcohol and the sugar alcohol includes sorbitol and mannitol, a weight ratio of an addition amount of the sorbitol to an addition amount of the mannitol is from 1:0.25 to 1:1.5; and when mixing the dihydroxyboryl compound and the sugar alcohol and the sugar alcohol includes sorbitol and xylitol, a weight ratio of an addition amount of the sorbitol to an addition amount of the xylitol is from 1:0.25 to 1:1.5.

In some embodiments, a pH value of the mixture is from 7.0 to 7.6.

In some embodiments, immersing the mixture into liquid nitrogen is performed for 5 minutes to 10 minutes.

In some embodiments, the vacuum drying process is performed at a room temperature in an environment with a pressure less than or equal to 1 Pa.

In some embodiments, the vacuum drying process is performed with a total time of 18 hours to 26 hours.

### BRIEF DESCRIPTION OF THE DRAWINGS

When reading the figures of the present disclosure, it is recommended to understand various aspects of the present disclosure from the detailed description provided below.
Fig. 1 is a schematic diagram of the flow chart of the method of forming the lyophilized powder containing boron complex according to some embodiments of the present disclosure.
Figs. 2A to 5B are spectra of the lyophilized powders containing boron complex measured by proton nuclear magnetic resonance according to some embodiments of the present disclosure.
Figs. 6A to 6D are spectra of the lyophilized powders containing boron complex measured by high-performance liquid chromatography (HPLC) according to some embodiments of the present disclosure.

### DETAILED DESCRIPTION

The present disclosure provides a lyophilized powder containing boron complex. The lyophilized powder includes a sugar acid and a complex formed by a dehydration condensation reaction of a dihydroxyboryl compound and a sugar or a sugar alcohol. The sugar acids may include sialic acid, neuraminic acid, glucuronic acid, mannuronic acid, or combinations thereof. The boron-containing complex in the lyophilized powder of the present disclosure can be used as the boron-containing drugs for, for example, the boron neutron capture therapy. In addition, the lyophilized powder has good solubility. For example, the lyophilized powder can be stored in a form of a lyophilized dry powder at a temperature at least from -20 °C to 25 °C, and when the lyophilized powder is dissolved in water, it can dissolve immediately within 1 minute, and the clear state of the solution lasts for at least 5 days. In addition, the boron-containing complex in the lyophilized powder of the present disclosure is stable and does not deteriorate when the lyophilized powder is stored. For example, the purity of the boron-containing complex after the lyophilized powder is stored and dissolved is detected to be more than 99% by high-performance liquid chromatography. In addition, the pH value of the lyophilized powder after it is dissolved corresponds to the range for the physiological applications. For example, the pH value of the lyophilized powder after it is dissolved is from 7.4 to 7.8, so the lyophilized powder can be used immediately as, for example, an intravenous fluid without further operations. Next, the lyophilized powder containing boron complex is described in detail according to some embodiments of the present disclosure.

First, the boron-containing complex is explained. The boron-containing complex includes the complex formed by the dehydration condensation reaction of the dihydroxyboryl compound with the sugar or the sugar alcohol. The boron-containing complex is formed by a dehydration condensation reaction on the dihydroxyboryl group (i.e., the borono group, the boric acid group, or 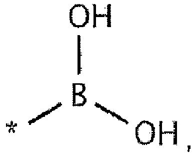 where * indicates a linking bond) of the dihydroxyboryl compound and the hydroxyl group of the sugar or the sugar alcohol. Since the sugar and the sugar alcohol can have more than one hydroxyl group bonding to the dihydroxyboryl compound (e.g., three hydroxyl groups, even though the present disclosure does not limit the number of the hydroxyl groups in the sugar and the sugar alcohol to bond to the dihydroxyboryl compound, and the complex of the present disclosure is intended to cover one, two, three, or combinations of the hydroxyl groups in the sugar or the sugar alcohol to bond to the dihydroxyboryl compound), the complex has a three-dimensional structure wrapping the water molecules or wrapped by the water molecules to improve the solubility of the boron-containing complex. In some embodiments, a first chemical shift of these complexes in the spectrum of proton nuclear magnetic resonance is from 7.30 ppm to 7.55 ppm, for example, 7.30 ppm, 7.35 ppm, 7.40 ppm, 7.45 ppm, 7.50 ppm, or 7.55 ppm, etc., and the number of peaks in this range may substantially be two or four. In some embodiments, a second chemical shift of these complexes in the spectrum of proton nuclear magnetic resonance is from 7.00 ppm to 7.19 ppm, for example, 7.00 ppm, 7.05 ppm, 7.10 ppm, 7.15 ppm, or 7.19 ppm, etc., and the number of peaks in this range may substantially be two.

Next, the dihydroxyboryl compound is explained. In addition to having a dihydroxyboronyl group to improve the solubility of the lyophilized powder by forming the complex, the dihydroxyboryl compound also has the structure, for example, a benzene ring, an amine group, and so on, to improve the affinity with the lesions when the dihydroxyboryl compound is used as, for example, the boron-containing drugs. In some embodiments, a preferable dihydroxyboryl compound includes boronophenylalanine (BPA), boronotryptophan (BT), 4-(benzo[d]thiazol-2-yl)phenylboronic acid (BTPB), or combinations thereof. The boronophenylalanine may have the structure of formula (1) (i.e., 4-BPA) or formula (2) (i.e., 3-BPA):

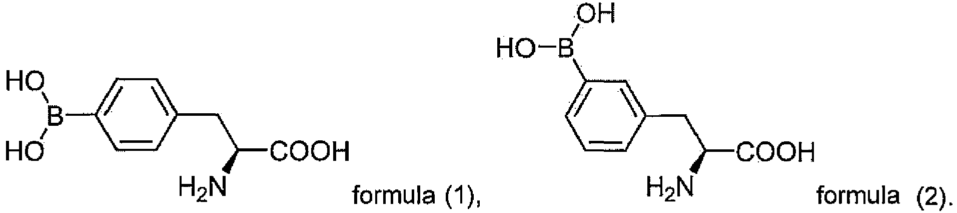

The boronotryptophan may have the structure of formula (3) (i.e., 6-BT) or formula (4) (i.e., 5-BT):

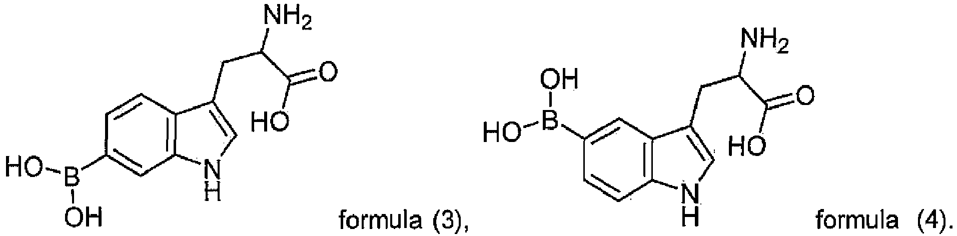

The 4-(benzo[d]thiazol-2-yl)phenylboronic acid may have the structure of formula (5). 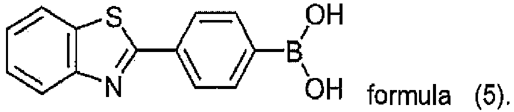 In some embodiments, the lyophilized powder further includes a dihydroxyboryl compound that is not formed in a complex with the sugar and the sugar alcohol. In some embodiments, a third chemical shift of the dihydroxyboryl compound that is not formed in a complex in the spectrum of proton nuclear magnetic resonance is from 7.56 ppm to 7.75 ppm, for example, 7.56 ppm, 7.60 ppm, 7.65 ppm, 7.70 ppm, or 7.75 ppm, etc., and the number of peaks in this range may substantially be two. In some embodiments, a fourth chemical shift of the dihydroxyboryl compound that is not formed in a complex in the spectrum of proton nuclear magnetic resonance is from 7.20 ppm to 7.29 ppm, etc., for example, 7.20 ppm, 7.25 ppm, or 7.29 ppm, and the number of peaks in this range may substantially be two. In some embodiments, a ratio of an integration of the first chemical shift of the complex to an integration of the third chemical shift of the dihydroxyboryl compound that is not formed in a complex in the spectrum of proton nuclear magnetic resonance spectrum is from 5 to 25, for example, 5, 8, 10, 12, 15, 18, 21, or 25, etc. The integration ratio of the proton signals is equivalent to the ratio of the amount of the protons in all the complexes to the amount of the protons in all the dihydroxyboryl compounds that do not form a complex in the lyophilized powder. When the integration ratio of the proton signals is in the range described above, the lyophilized powder has good solubility and is also effective to act as a boron-containing drug. In some embodiments, more than at least 20% of the boron atoms in the dihydroxyboryl compounds and the complexes including the dihydroxyboryl compounds are ¹⁰B.

Next, the sugar and the sugar alcohol are explained. In some embodiments, the sugar includes fructose, mannose, or a combination thereof. Taking the 4-BPA as an example, the complex formed by the 4-BPA and the fructose may have the structure of the formula (6): 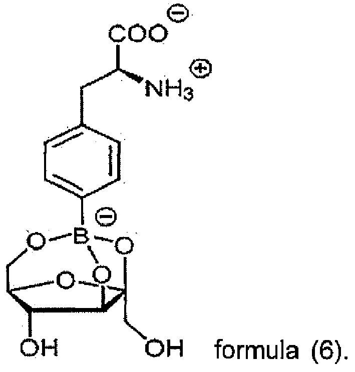 In some embodiments, the sugar alcohol includes sorbitol, xylitol, mannitol, or combinations thereof. Taking the 4-BPA as an example, the complex formed by the 4-BPA and the sorbitol may have the structure of the formula (7): 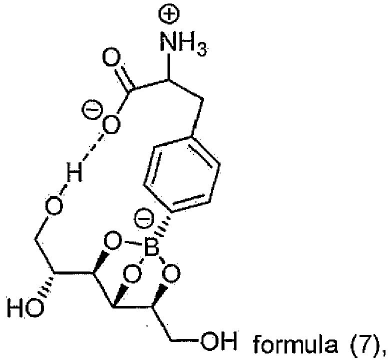 where the dashed line between O and H is denoted as a hydrogen bond. Taking the 4-BPA as an example, the complex formed by the 4-BPA and the xylitol may have the structure of the formula (8): 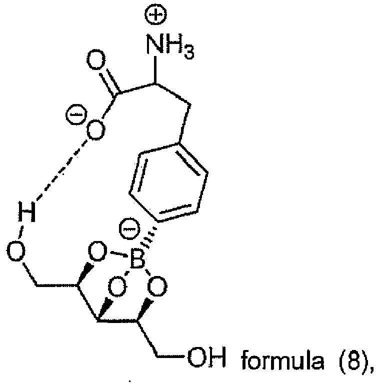 where the dashed line between O and H is denoted as a hydrogen bond. In some embodiments, a preferable sugar alcohol includes the combination of the sorbitol and the xylitol such that the complex includes a first complex formed by the dihydroxyboryl compound and the sorbitol (e.g., the complex including the formula (7) shown above) and a second complex formed by the dihydroxyboryl compound and the xylitol (e.g., the complex including the formula (8) shown above), so the lyophilized powder can be dissolved more quickly to become a clear solution and the clear state of the solution can last for a longer time. In some embodiments, the molar ratio of the first complex to the second complex in the lyophilized powder is preferably from 1:0.29 to 1:1.8, for example, 1:0.29, 1:0.6, 1:0.9, 1:1.2, 1:1.5, or 1:1.8, etc. In some embodiments, a preferable sugar alcohol includes the combination of the sorbitol and the mannitol such that the complex includes the first complex formed by the dihydroxyboryl compound and the sorbitol (e.g., the complex including the formula (7) shown above) and a third complex formed by the dihydroxyboryl compound and the mannitol (not additionally drawn herein), so the lyophilized powder can be dissolved more quickly to become a clear solution and the clear state of the solution can last for a longer time. In some embodiments, the molar ratio of the first complex to the third complex in the lyophilized powder is preferably from 1:0.25 to 1:1.5, for example, 1:0.25, 1:0.5, 1:0.75, 1:1, 1:1.25, or 1:1.5, etc.

Next, the sugar acid is explained. The sugar acid includes sialic acid (e.g., N-acetylneuraminic acid, 2-keto-3-deoxynonic acid, the analogue, or combinations thereof), neuraminic acid, glucuronic acid, mannuronic acid, or combinations thereof, which are the compounds derived from sugars and have carboxyl groups. The sugar acid can have hydrogen bonding with the complex formed by the dihydroxyboryl compound and the sugar or the sugar alcohol and/or have hydrogen bonding with the dihydroxyboryl compound that is not formed in a complex, thus improving the solubility of the lyophilized powder further. Taking the 4-BPA as an example, the hydrogen bonding between the 4-BPA and the sialic acid is confirmed by a theoretical calculation to have the structure as the following formula (9): 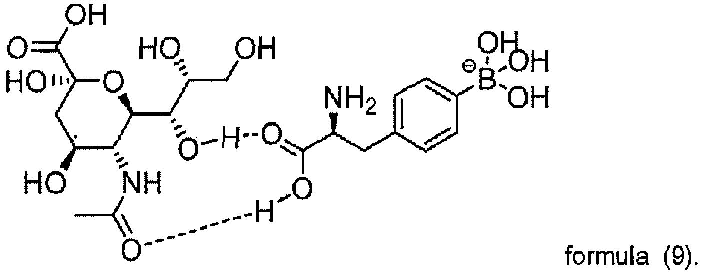 In addition, the sugar acid can also adjust the pH value of the lyophilized powder. In some embodiments, the pH value of the lyophilized powder after dissolving in water is preferably from 7.4 to 7.8, for example, 7.4, 7.5, 7.6, 7.7, or 7.8, etc.

The lyophilized powder of the present disclosure is formed by freezing and sublimation drying (please refer to the method described below for more details), so the lyophilized powder has a certain degree of crystallization. In some embodiments, the lyophilized powder is a fluffy solid, and the particle of the lyophilized powder is full and does not collapse. In some embodiments, the particle size of the lyophilized powder (measured by high-resolution thermal field emission scanning electron microscopy) is preferably from 50 µm to 140 µm, for example, 50 µm, 80 µm, 110 µm, or 140 µm, etc.

The present disclosure also provides a method of forming the lyophilized powder containing boron complex described above. The method includes the following operations. The dihydroxyboryl compound and the sugar or the sugar alcohol are mixed to form a mixture, in which the mixture includes the complex formed by a dehydration condensation reaction of the dihydroxyboryl compound and the sugar or the sugar alcohol. The mixture is immersed into liquid nitrogen to freeze the mixture to form a pre-frozen body. A vacuum drying process is performed to evaporate water from the pre-frozen body to form the lyophilized powder. The advantageous effects of the lyophilized powder formed by the method of the present disclosure can be referred to above. In addition, the method of the present disclosure is simple, fast, and easy to implement, thus reducing costs and increasing productivity. Next, the method is described in detail according to some embodiments of the present disclosure.

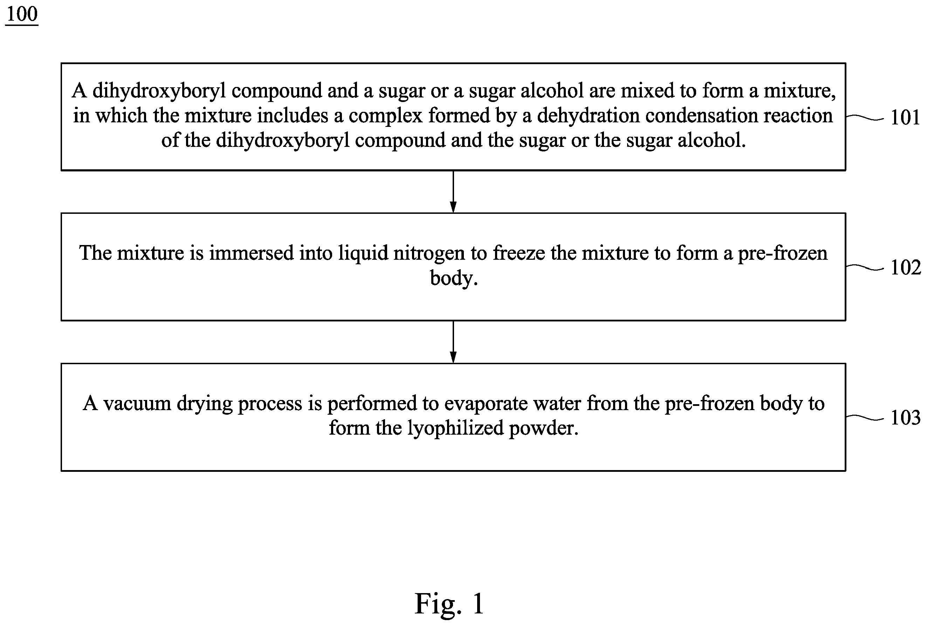

Referring to the operation 101 in the method 100 of Fig. 1, the dihydroxyboryl compound and the sugar or the sugar alcohol are mixed to form a mixture including the complex formed by the dehydration condensation reaction of the dihydroxyboryl compound with the sugar or the sugar alcohol. In some embodiments, mixing the dihydroxyboryl compound and the sugar or the sugar alcohol further includes mixing the sugar acid, in which the sugar acid includes sialic acid, neuraminic acid, glucuronic acid, mannuronic acid, or combinations thereof, and a weight ratio of the addition amount of the sugar alcohol to the addition amount of the sugar acid may be from 2:1 to 7:1, for example, 2:1, 3:1, 4:1, 5:1, 6:1, or 7:1, etc. The description of the dihydroxyboryl compound, the sugar, the sugar alcohol, and the sugar acid can refer to the description above. In some embodiments, the dihydroxyboryl compound is mixed with the sugar or the sugar alcohol in an alkaline aqueous solution before the acidic substance including the sugar acid is added to the alkaline aqueous solution for further mixing, so the boron atom in the dihydroxyboryl group of the dihydroxyboryl compound can bond with OH- in the alkaline aqueous solution to have a negative charge in the dihydroxyboryl compound, which the structure facilitates the formation of the complex. After the complex is formed, the solution is adjusted to a desired pH value by adding the acidic substance. Taking the reaction formula below as an example, the 4-BPA can form the structure (the structure on the right side of the reaction formula) with a negative charge on the boron atom in an alkaline aqueous solution, and this structure can further form the complex of the present disclosure (e.g., the structure of the formula (6), formula (7), or formula (8) shown above): 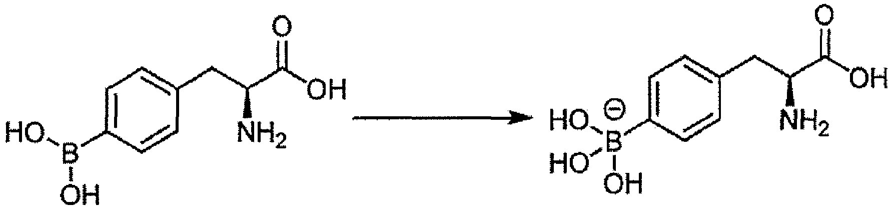 In some embodiments, the pH value of the alkaline aqueous solution is preferably from 8.3 to 8.7, for example, 8.3, 8.4, 8.5, 8.6, or 8.7, etc. In some embodiments, the alkaline aqueous solution is an aqueous solution of sodium hydroxide. In some embodiments, in addition to the sugar acid, the acidic substance may further include hydrochloric acid, for example, 1N hydrochloric acid, 3N hydrochloric acid, 6N hydrochloric acid, or combinations thereof. In some embodiments, a high concentration (e.g., 6N) of hydrochloric acid is added followed by the sugar acid or a combination of the sugar acid and a low concentration (e.g., 3N) of hydrochloric acid, to more accurately adjust the pH value of the mixture. In some embodiments, the pH value of the mixture is preferably from 7.0 to 7.6, for example, 7.0, 7.1, 7.2, 7.3, 7.4, 7.5, or 7.6, etc. In some embodiments, a vortex vibrator may be used to assist in mixing the dihydroxyboryl compound with the sugar or the sugar alcohol (or further with the sugar acid).

Continue to explain the operation 101. In some embodiments, when mixing the dihydroxyboryl compound and the sugar or the sugar alcohol, the equivalent ratio of the addition amount of the sugar or the sugar alcohol to the addition amount of the dihydroxyboryl compound is preferably from 1.0 to 1.5, for example, 1.0, 1.1, 1.2, 1.3, 1.4, or 1.5, etc. In some embodiments, when the dihydroxyboryl compound and the sugar alcohol are mixed and the sugar alcohol includes the sorbitol and the xylitol (hence, the lyophilized powder including the complexes formed by the sorbitol and the xylitol), the weight ratio of the addition amount of the sorbitol to the addition amount of the xylitol is preferably from 1:0.25 to 1:1.5, for example, 1:0.5, 1:0.75, 1:1, 1:1.25, or 1:1.5, etc., so the lyophilized powder dissolves clearly in the solution more quickly and stays in a clear state for a long time. In some embodiments, when the dihydroxyboryl compound and the sugar alcohol are mixed and the sugar alcohol includes the sorbitol and the mannitol (hence, the lyophilized powder including the complexes formed by the sorbitol and the mannitol), the weight ratio of the addition amount of the sorbitol to the addition amount of the mannitol is preferably from 1:0.25 to 1:1.5, for example, 1:0.5, 1:0.75, 1:1, 1:1.25, or 1:1.5, etc., so the lyophilized powder dissolves clearly in the solution more quickly and stays in a clear state for a long time. In some embodiments, the operation 101 further includes filtering the mixture with a membrane with a pore size of about 0.22 µm to remove the bacteria (e.g., endotoxin, etc.) in the mixture after the mixture has been formed.

Referring to the operation 102 in the method 100 of Fig. 1, the mixture obtained from the operation 101 is immersed into liquid nitrogen to rapidly freeze the mixture and prepare the mixture to be a pre-frozen body for the use in the operation 103. In some embodiments, since the liquid nitrogen has an extremely low temperature of about -196 °C, the immersion process of the operation 102 takes only 5 minutes to 10 minutes, for example, 5 minutes, 6 minutes, 7 minutes, 8 minutes, 9 minutes, or 10 minutes, etc. The composition of the pre-frozen body obtained after the completion of the operation 102 is substantially the same as the composition of the mixture, except that the pre-frozen body is in a crystalline state and the temperature of the pre-frozen body is below at least -170 °C. The operation 102 does not contaminate the composition of the mixture and the pre-frozen body, does not damage the mixture and the pre-frozen body, and does not make the mixture and the pre-frozen body decompose and/or convert into other components. For example, the purity of the complex in the lyophilized powder is high (e.g., up to 99%) by the measurement of high-performance liquid chromatography, and no corresponding peaks of, for example, phenylalanine, tyrosine, etc., generated from the decomposition and/or conversion of the complex are found.

Referring to the operation 103 in the method 100 of Fig. 1, a vacuum drying process is performed to volatilize the water in the pre-frozen body obtained from the operation 102 such that the water can completely desorb from the pre-frozen body to form the lyophilized powder. The detailed description of the lyophilized powder has been provided above. In some embodiments, the operation 103 is performed directly after the completion of the operation 102 without other operations in between and waiting time. That is, the time interval between the operation 102 and the operation 103 is substantially equal to zero except for the time lag caused by the actual operation. In some embodiments, the vacuum drying process is performed in a process environment with a pressure of less than or equal to 1 Pa and greater than 0 Pa, for example, 0.05 Pa to 1 Pa, e.g., 0.05 Pa, 0.1 Pa, 0.11 Pa, 0.5 Pa, or 1 Pa, etc. In some embodiments, the vacuum drying process is performed at room temperature, for example, 20 °C to 30 °C, e.g., 20 °C, 22 °C, 24 °C, 26 °C, 28 °C, or 30 °C, etc. In some embodiments, the total process time of the vacuum drying process is from 18 hours to 26 hours, for example, 18 hours, 19 hours, 20 hours, 21 hours, 22 hours, 24 hours, or 26 hours, etc. In some embodiments, the yield of the lyophilized powder formed by performing the vacuum drying process is from 50% to 80%. In some embodiments, the vacuum drying process of forming the lyophilized powder is a single continuous and uninterrupted process. The operation 103 not only volatilizes the water, but also completely desorbs the water from the pre-frozen body, so the present disclosure does not require performing additional desorption-related drying processes after the completion of the operation 103. In some embodiments, the operation 103 further includes grinding the lyophilized powder formed after the vacuum drying process to form the lyophilized powder with the desired particle size. After the completion of the operation 103, the lyophilized powder can be stored directly at a temperature at least from -20 °C to 25 °C (e.g., -20 °C, -10 °C, 0 °C, 4 °C, 10 °C, 20 °C, or 25 °C, etc.) without performing other operations, and the lyophilized powder does not deteriorate easily in this range. Moreover, the lyophilized powder does not deteriorate even when the lyophilized powder is stored at a temperature at least from -20 °C to 20 °C for at least 3 years.

Next, the lyophilized powder and the method of the present disclosure are explained according to some detailed embodiments. It should be noted that the following detailed embodiments are intended to make the present disclosure more understandable to one skilled in the art, and are not intended to limit the scope of the present disclosure.

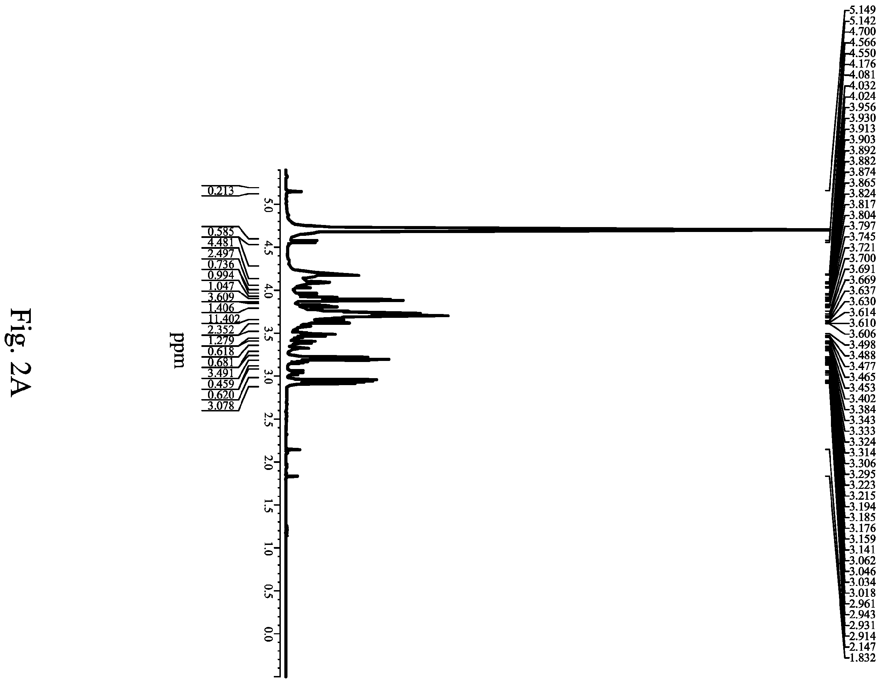

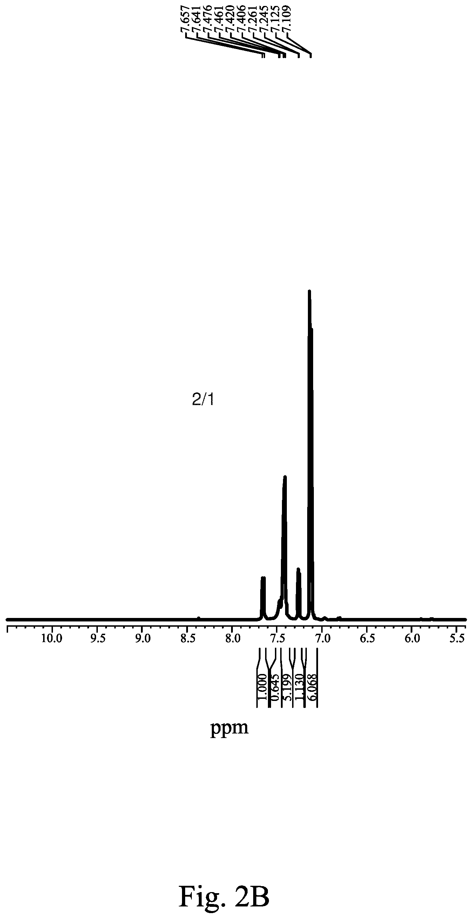

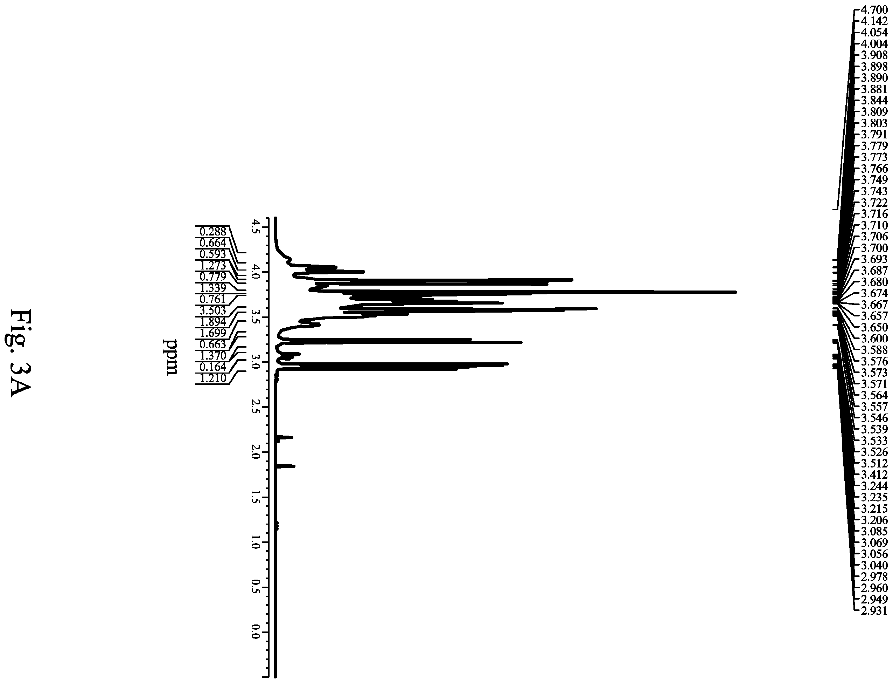

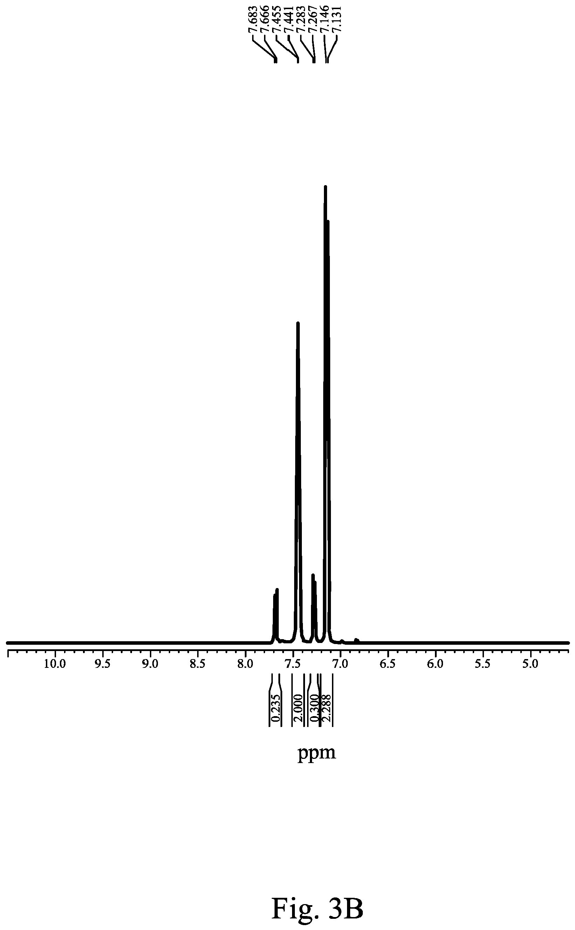

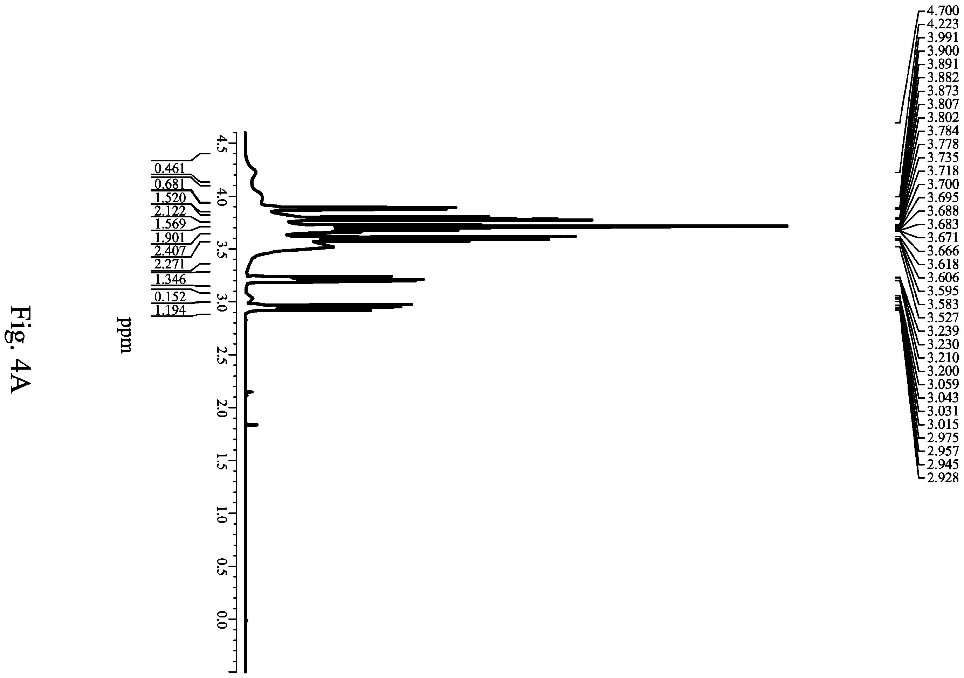

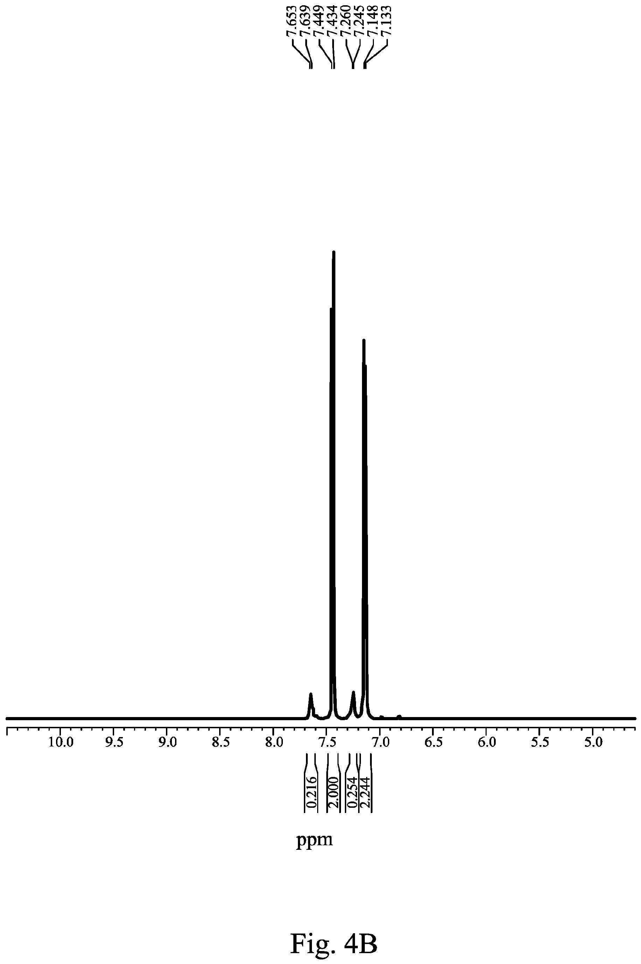

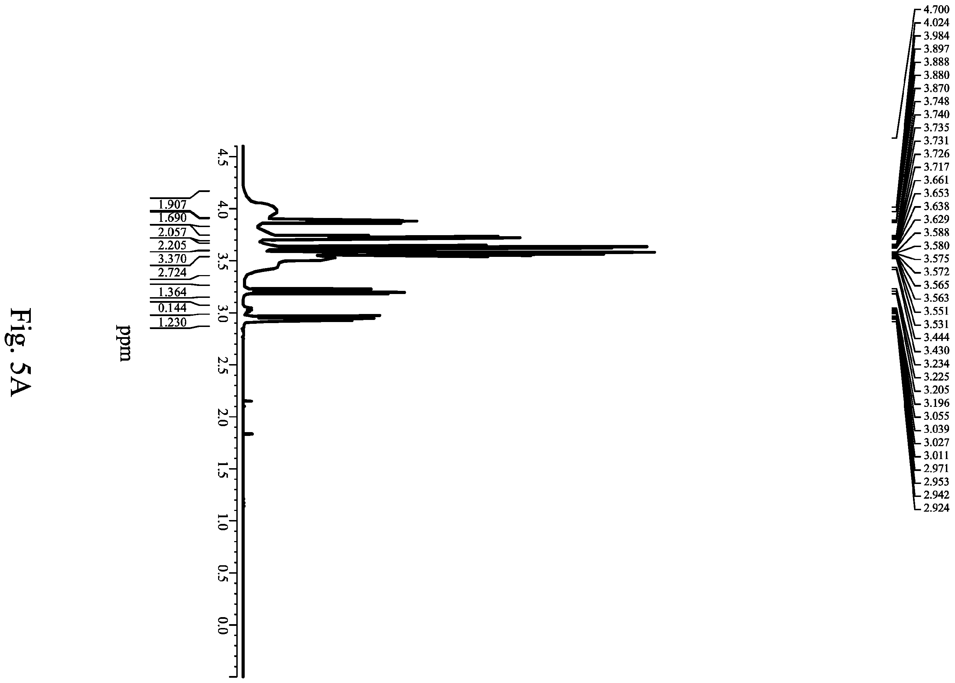

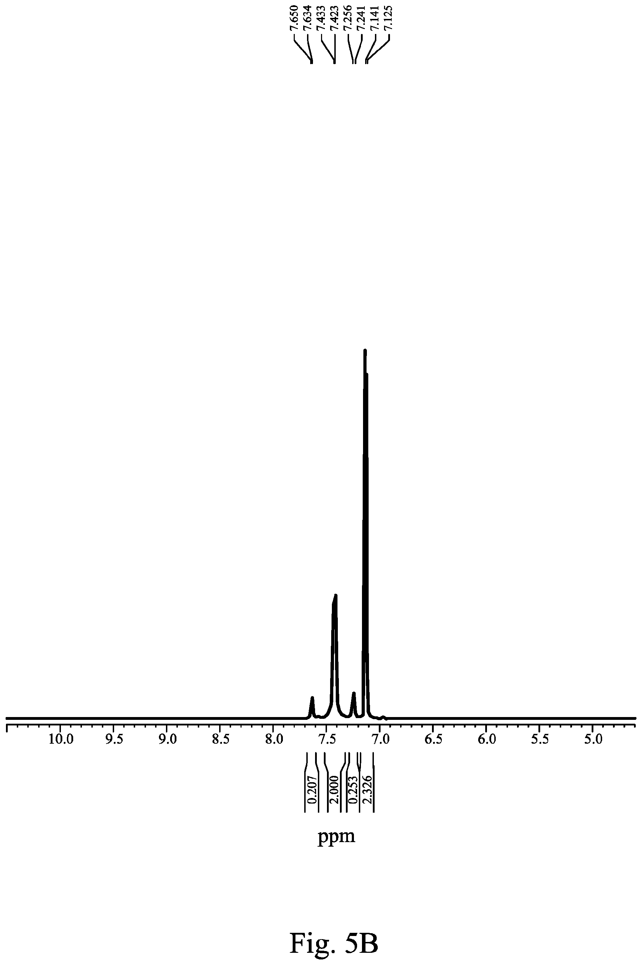

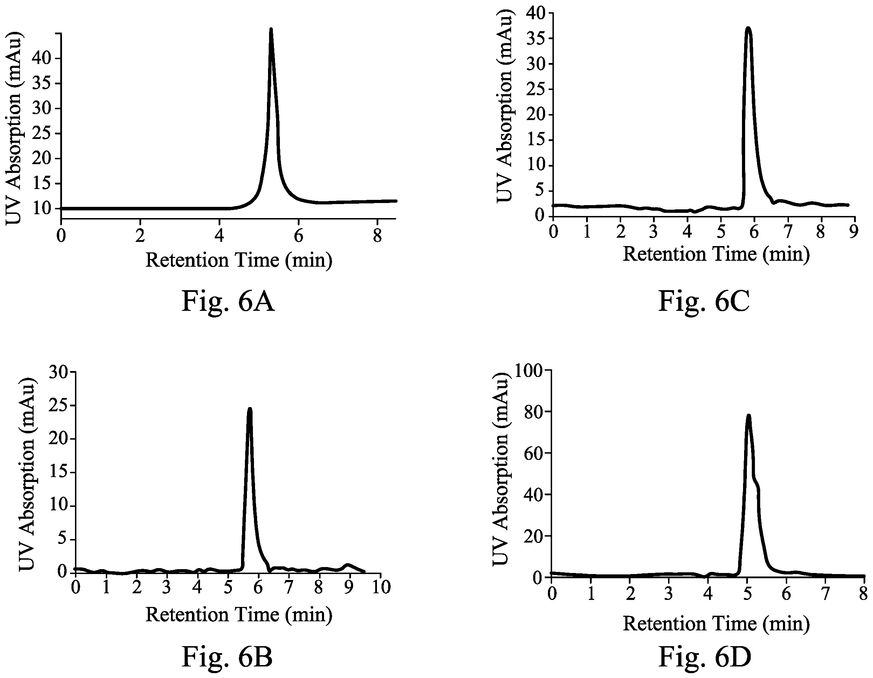

In the embodiment E1, the embodiment E2, the embodiment E3, the embodiment E4, the embodiment E5, the embodiment E6, the embodiment E7, the embodiment E8, and the embodiment E9, 1 mL of 0.15 N sodium hydroxide aqueous solution was drawn twice with a pipette into a 5 mL Eppendorf Tube^{®}, and then 60 mg of the 4-BPA and 64 mg of the sugar or the sugar alcohol (please referring to Table 1 for the actual composition in each embodiment) were added into the Eppendorf Tube^{®}. Then, a vortex vibrator was used for 10 minutes to assist in mixing the 4-BPA and the sugar or the sugar alcohol to form a clear solution. Then, 5 µL to 8 µL of 6N hydrochloric acid and 2 µL to 5 uL of the combination of the sialic acid and 3N hydrochloric acid were added into the Eppendorf Tube^{®} to adjust the pH value of the mixture according to the requirement of each embodiment (please referring to Table 1 for the actual pH value of the mixture in each embodiment). The mixture was then filtered with a membrane of 0.22 µm pore size and transferred into an Eppendorf tube^{®} that was next immersed into a dewar including the liquid nitrogen for 5 minutes to 10 minutes to obtain the pre-frozen body. The pre-frozen body was then vacuum dried at a pressure of 0.1 Pa at 20 °C for 18 hours and at 25 °C for 6 hours to form the lyophilized powder. Then, after being stored at a temperature of about -20 °C for about 1 month, the lyophilized powder was dissolved in water. The result showed that the lyophilized powders in the embodiment E1 to the embodiment E9 dissolved into water quickly and a clear state of the solution stayed for a long time. The integration ratio of the proton signals between the first chemical shift of the complex and the third chemical shift of the 4-BPA that does not form a complex was measured by proton nuclear magnetic resonance, and the result showed that the integration ratios in these embodiments were quite high to correspond to the good solubility of the lyophilized powders. The first chemical shifts and the corresponding integration values of the complexes, the third chemical shifts and the corresponding integration values of the 4-BPAs that do not form the complex, and the integration ratios of the first chemical shifts to the third chemical shifts in the embodiment E1 to the embodiment E9 are summarized in Table 1. The sections of the spectra measured by proton nuclear magnetic resonance in the embodiment E1, the embodiment E4, the embodiment E8, and the embodiment E9 are respectively provided in Figs. 2A-2B, Figs. 3A-3B, Figs. 4A-4B, and Figs. 5A-5B, and the spectra of the remaining embodiments are not provided to simplify the present disclosure. In addition, after the lyophilized powders were dissolved, the peaks of the complexes in the embodiment E1 to the embodiment E9 did not show splitting or impurities when measured by high-performance liquid chromatography, which indicates that the lyophilized powders have high stability and can be stored for a long time without deterioration. The spectra measured by high-performance liquid chromatography in the embodiment E3, the embodiment E8, and the embodiment E9 are respectively provided in Fig. 6A, Fig. 6B, and Fig. 6C, and the spectra of the remaining embodiments are not provided to simplify the present disclosure.

**Table 1 (the lyophilized powders were stored at a temperature of about -20 °C for about 1 month)**

| | Dihydroxyboryl Compound | sugar or Sugar Alcohol | pH | Chemical Shift of Complex | Integration of Complex | Chemical Shift of Dihydroxyboryl Compound | Integration of Dihydroxyboryl Compound | Integration Ratio |
|---|---|---|---|---|---|---|---|---|
| E1 | 4-BPA | fructose | 7.2 | 7.476, 7.461, 7.420, 7.406 | 5.844 | 7.657, 7.641 | 1.000 | 5.844 |
| E2 | 4-BPA | fructose | 7.4 | 7.475, 7.460, 7.418, 7.404 | 5.917 | 7.639, 7.624 | 1.000 | 5.917 |
| E3 | 4-BPA | fructose | 7.6 | 7.474, 7.460, 7.416, 7.403 | 5.920 | 7.628, 7.614 | 1.000 | 5.920 |
| E4 | 4-BPA | sorbitol | 7.0 | 7.455, 7.441 | 2.000 | 7.683, 7.666 | 0.207 | 9.66 |
| E5 | 4-BPA | sorbitol | 7.2 | 7.455, 7.441 | 2.000 | 7.682, 7.667 | 0.170 | 11.76 |
| E6 | 4-BPA | sorbitol | 7.4 | 7.454, 7.440 | 2.000 | 7.669, 7.654 | 0.102 | 19.61 |
| E7 | 4-BPA | sorbitol | 7.6 | 7.454, 7.439 | 2.000 | 7.659, 7.644 | 0.107 | 18.69 |
| E8 | 4-BPA | mannitol | 7.6 | 7.449, 7.434 | 2.000 | 7.653, 7.639 | 0.216 | 9.26 |
| E9 | 4-BPA | xylitol | 7.6 | 7.433, 7.423 | 2.000 | 7.650, 7.634 | 0.207 | 9.66 |

The operations of the embodiment E10, the embodiment E11, the embodiment E12, and the embodiment E13 were substantially the same as those of the embodiment E1 to the embodiment E9, except that the lyophilized powders were dissolved in water after being stored at a temperature of 25 °C for about 1 month, and the corresponding parameters of the embodiment E10 to the embodiment E13 please refer to Table 2. The result showed that the lyophilized powders in the embodiment E10 to the embodiment E13 also dissolved into water quickly and a clear state of the solution stayed for a long time. The integration ratios of the proton signals between the first chemical shifts of the complex and the third chemical shifts of the 4-BPAs that do not form the complexes, measured by proton nuclear magnetic resonance, were also quite high in these embodiments to correspond to the good solubility of the lyophilized powders. The spectra measured by proton nuclear magnetic resonance in these embodiments are not provided to simplify the present disclosure. In addition, after the lyophilized powders were dissolved, the peaks of the complexes in the embodiment E10 to the embodiment E13 also did not show splitting or impurities when measured by high-performance liquid chromatography, which indicates that the lyophilized powders have high stability and can be stored for a long time without deterioration. The spectrum measured by high-performance liquid chromatography in the embodiment E12 is provided in Fig. 6D, and the spectra of the remaining embodiments are not provided to simplify the present disclosure.

**Table 2 (the lyophilized powders were stored at a temperature of about 25 °C for about 1 month)**

| | Dihydroxyboryl Compound | sugar or Sugar Alcohol | pH | Chemical Shift of Complex | Integration of Complex | Chemical Shift of Dihydroxyboryl Compound | Integration of Dihydroxyboryl Compound | Integration Ratio |
|---|---|---|---|---|---|---|---|---|
| E10 | 4-BPA | sorbitol | 7.0 | 7.453, 7.439 | 2.000 | 7.682, 7.666 | 0.185 | 10.81 |
| E11 | 4-BPA | sorbitol | 7.2 | 7.453, 7.439 | 2.000 | 7.678, 7.663 | 0.170 | 11.76 |
| E12 | 4-BPA | sorbitol | 7.4 | 7.453, 7.439 | 2.000 | 7.669, 7.654 | 0.098 | 20.41 |
| E13 | 4-BPA | sorbitol | 7.6 | 7.451, 7.437 | 2.000 | 7.653, 7.638 | 0.100 | 20.00 |

The similar operations of the embodiment E1 to the embodiment E13 were also repeated to form larger amounts of the lyophilized powders, and the results showed that the lyophilized powders had good solubility and were also very stable. Specifically, to form larger amounts of the lyophilized powders, 2 mL of 0.15 N sodium hydroxide aqueous solution was changed to 34 mL of 0.15 N sodium hydroxide aqueous solution, 5 mL of Eppendorf Tube^{®} was changed to 50 mL beaker, 60 mg of the 4-BPA was changed to 1.2 g of the 4-BPA, 64 mg of the sugar or the sugar alcohol shown in Table 1 and Table 2 was changed to 1.28 g of the corresponding sugar or sugar alcohol, and 6N hydrochloric acid and the combination of the sialic acid and 3N hydrochloric acid were adjusted accordingly to provide the pH value shown in Table 1 and Table 2, etc.. The remaining operations are substantially the same as those shown in the embodiment E1 to the embodiment E13.

The lyophilized powder of the present disclosure is in the form of dry powder, so the weight and volume to package the lyophilized powder can be reduced, and the preservation, transportation, and management of the lyophilized powder are easier to implement. The lyophilized powder of the present disclosure also has good solubility and stability. For example, the lyophilized powder can be stored from low temperature to room temperature for a long time without deterioration, the lyophilized powder can be quickly dissolved when it is dissolved in water, and the clear state of the solution after the lyophilized powder is dissolved can last for a long time. The pH value of the lyophilized powder of the present disclosure after it is dissolved is in the range for the physiological application, so the lyophilized powder is more convenient to be used as a boron-containing drug. In addition, the method of the present disclosure is simple, is fast, is easy to implement, has high yield, has high purity, and can be manufactured in large quantities to reduce the cost.

## Claims

1. A lyophilized powder containing boron complex, **characterized by** comprising:
a sugar acid; and
a complex formed by a dehydration condensation reaction of a dihydroxyboryl compound and a sugar or a sugar alcohol.

2. The lyophilized powder of claim 1, **characterized in that** the sugar acid comprises sialic acid, neuraminic acid, glucuronic acid, mannuronic acid, or combinations thereof.

3. The lyophilized powder of any one of claims 1 to 2, **characterized in that** the sugar alcohol comprises sorbitol, mannitol, xylitol, or combinations thereof.

4. The lyophilized powder of any one of claims 1 to 3, **characterized in that** the complex comprises a first complex formed by the dihydroxyboryl compound and sorbitol and a second complex formed by the dihydroxyboryl compound and mannitol or xylitol.

5. The lyophilized powder of any one of claims 1 to 4, **characterized by** further comprising a dihydroxyboryl compound that is not formed in a complex, and in a spectrum of a proton nuclear magnetic resonance, a ratio of an integration of a signal of the complex to an integration of a signal of the dihydroxyboryl compound that is not formed in a complex is from 5 to 25.

6. The lyophilized powder of claim 5, **characterized in that** the signal of the complex is from 7.30 ppm to 7.55 ppm, and the signal of the dihydroxyboryl compound that is not formed in a complex is from 7.56 ppm to 7.75 ppm.

7. The lyophilized powder of any one of claims 1 to 6, **characterized in that** a pH value of the lyophilized powder dissolved in water is from 7.4 to 7.8.

8. The lyophilized powder of any one of claims 1 to 7, **characterized in that** a particle size of the lyophilized powder is from 50 µm to 140 µm.

9. A method of forming a lyophilized powder containing boron complex, **characterized by** comprising:
mixing a dihydroxyboryl compound and a sugar or a sugar alcohol to form a mixture, wherein the mixture comprises a complex formed by a dehydration condensation reaction of the dihydroxyboryl compound and the sugar or the sugar alcohol;
immersing the mixture into liquid nitrogen to freeze the mixture to form a pre-frozen body; and
performing a vacuum drying process to evaporate water from the pre-frozen body to form the lyophilized powder.

10. The method of claim 9, **characterized in that** mixing the dihydroxyboryl compound and the sugar or the sugar alcohol further comprises mixing a sugar acid to form the mixture, wherein the sugar acid comprises sialic acid, neuraminic acid, glucuronic acid, mannuronic acid, or combinations thereof.

11. The method of any one of claims 9 to 10, **characterized in that** mixing the dihydroxyboryl compound and the sugar or the sugar alcohol further comprises mixing a sugar acid to form the mixture, the dihydroxyboryl compound and the sugar or the sugar alcohol are mixed in an alkaline aqueous solution before adding the sugar acid, and a pH value of the alkaline aqueous solution is from 8.3 to 8.7.

12. The method of any one of claims 9 to 11, **characterized in that** when mixing the dihydroxyboryl compound and the sugar or the sugar alcohol, an equivalent ratio of an addition amount of the sugar or the sugar alcohol to an addition amount of the dihydroxyboryl compound is from 1.0 to 1.5.

13. The method of any one of claims 9 to 12, **characterized in that** when mixing the dihydroxyboryl compound and the sugar alcohol and the sugar alcohol comprises sorbitol and mannitol, a weight ratio of an addition amount of the sorbitol to an addition amount of the mannitol is from 1:0.25 to 1:1.5; and when mixing the dihydroxyboryl compound and the sugar alcohol and the sugar alcohol comprises sorbitol and xylitol, a weight ratio of an addition amount of the sorbitol to an addition amount of the xylitol is from 1:0.25 to 1:1.5.

14. The method of any one of claims 9 to 13, **characterized in that** a pH value of the mixture is from 7.0 to 7.6.

15. The method of any one of claims 9 to 14, **characterized in that** the vacuum drying process is performed at a room temperature in an environment with a pressure less than or equal to 1 Pa.
